# EUROPEAN PATENT APPLICATION

(11) **EP 1 625 875 A1**
(43) Date of publication of application: **15.02.2006**
(21) Application number: 05254957.3
(22) Date of filing: 09.08.2005
(51) Int. Cl.: A61N 1/375

(54) **System and method for providing strain relief for medical device leads**

(30) Priority: 09.08.2004 US 599953 P; 08.08.2005 US 199048
(71) Applicant: ADVANCED NEUROMODULATION SYSTEMS, INC., Plano, TX 75024 (US)
(72) Inventor: Jones, Robert E., McKinney Texas 75069 (US); Cullen, Patrick M., Dallas Texas 75229 (US); Daglow, Terry D., Allen Texas 75013 (US)
(74) Representative: Jackson, Richard Eric

(57) **Abstract**

Disclosed are systems and methods which provide a header assembly for interfacing a medical device with a lead, wherein the header assembly includes a strain relief portion integral thereto. The strain relief portion may be adapted to facilitate a user visually verifying when a lead has been fully inserted into the header. Structure, such as a band or ring, may be provided upon the strain relief portion in order to provide a readily identifiable visual aid for determining when a lead is fully inserted.

## Description

### RELATED APPLICATIONS

Priority is hereby claimed to co-pending and commonly assigned United States provisional patent application serial number 60/599,953 entitled "System and Method for Providing Strain Relief for Medical Device Leads," filed August 9, 2004, the disclosure of which is hereby incorporated herein by reference. The present application is related to co-pending and commonly assigned United States patent applications serial number 29/206,769 entitled "Design for Stimulator Lead Adapter," filed June 3, 2004, and serial number 29/204,866 entitled "Stimulator Lead Adapter," filed May 5, 2004, the disclosures of which are hereby incorporated herein by reference.

### TECHNICAL FIELD

The present invention relates generally to medical devices having leads associated therewith and, more particularly, to providing strain relief with respect to the foregoing leads.

### BACKGROUND OF THE INVENTION

Numerous medical devices have been developed and utilized for providing pain management therapy to patents. For example, United States patent numbers 5,938,690, 6,027,456, and 6,609,031, the disclosures of which are incorporated herein by reference, show neurostimulation systems in which a pulse generator and electrical lead are implanted in a patient to provide pain management through controlled electro-stimulation.

Generally a strain relief mechanism is employed with respect to the aforementioned electrical lead at a point where the lead attaches to a pulse generator in order to prevent the lead from being tightly radiused (i.e., "kinked") or otherwise having excess strain exerted thereon. Heretofore, such strain relief has been provided by a strain relief member which is a discrete component separate from the lead and the pulse generator or by a strain relief component which is integral with a lead plug assembly.

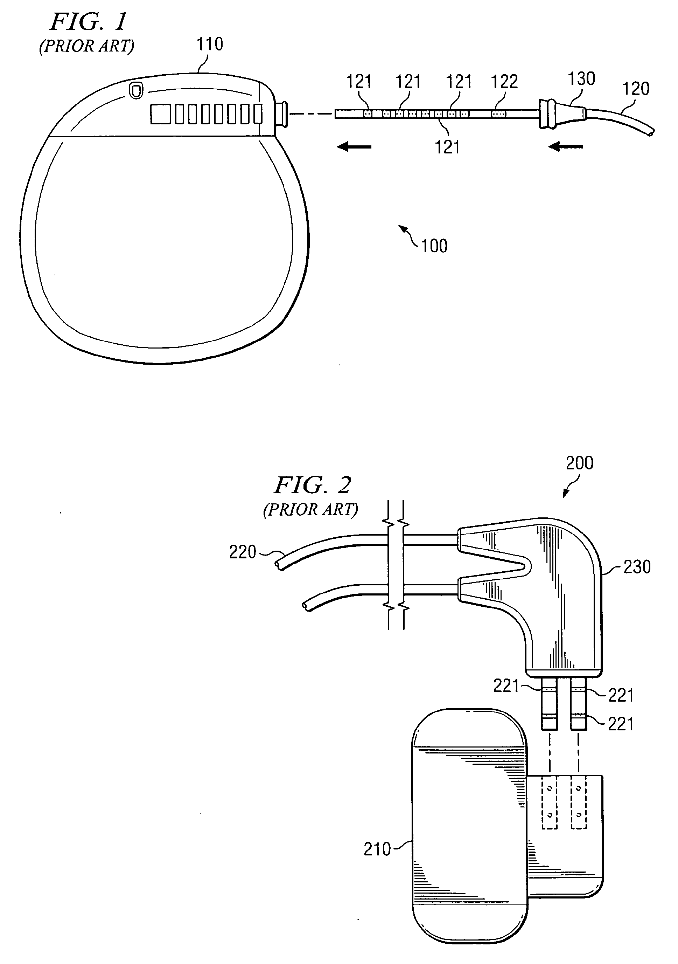

Directing attention to FIGURE 1, a prior art configuration in which a strain relief member is provided separate from the lead and pulse generator is shown. Specifically, neurostimulation system 100 includes implantable pulse generator 110 and flexible lead 120. An end of lead 120, having electrodes 121 sized and shaped to correspond to electrical contacts disposed within pulse generator 110, is inserted into pulse generator 110 during installation to provide an electrical conduit through which therapeutic stimulation pulses may pass for delivery to the patient. Strain relief member 130 is provided for interfacing with both pulse generator 110 and lead 120, when lead 120 is fully inserted into pulse generator 110, to thereby provide strain relief at the interface of pulse generator 110 and lead 120. In order to be properly installed, strain relief member 130 must be placed on lead 120 in the proper orientation prior to insertion of lead 120 into pulse generator 110. Thereafter, lead 120 is inserted into a lead orifice of pulse generator 110. In order to assist in the insertion of lead 120, insertion handle 122 may be included on lead 120 to provide a relatively rigid portion of an otherwise flexible lead, and thus facilitate pushing of an end of lead 120 into the lead orifice of pulse generator 110. Insertion handle 122 may be obscured from view by a portion of pulse generator 110 when lead 120 is fully inserted to provide a visual reference to the installer. Once lead 120 is fully inserted into pulse generator 110, strain relief member 130 may be slid along lead 120 to engage pulse generator 110. Strain relief member 130 would typically be sutured in place once engaged with pulse generator 110 to prevent its becoming displaced and thus reducing or negating its effectiveness at providing desired strain relief. Although providing a relatively low cost and effective strain relief, the configuration of FIGURE 1 provides opportunity for error during installation.

FIGURE 2 shows another prior art strain relief configuration. In the configuration of FIGURE 2, a strain relief component is integral with a rigid lead plug assembly. Specifically, neurostimulation system 200 includes implantable pulse generator 210 and lead assembly 220. An end of lead assembly 220, having a rigid lead plug assembly with electrodes 221 sized and shaped to correspond to electrical contacts disposed within pulse generator 210, is inserted into pulse generator 210 during installation to provide an electrical conduit through which therapeutic stimulation pulses may pass for delivery to the patient. Strain relief portion 230 is provided integral with lead assembly 220 at a point which the flexible lead interfaces with the rigid lead plug to thereby provide strain relief for the flexible lead at the interface of pulse generator 210 and lead assembly 220. Although providing strain relief which is very simple to implement in the field, the configuration of FIGURE 2 requires complicated and costly manufacturing techniques and results in a large lead assembly and pulse generator lead interface.

A need exists in the art for effective and relatively inexpensive strain relief which may be implemented in the field with little difficulty.

### BRIEF SUMMARY OF THE INVENTION

The present invention is directed to systems and methods which provide a header assembly for interfacing a pulse generator or other medical device with a lead, wherein the header assembly includes a strain relief portion integral thereto. A header assembly having integrated strain relief according to an embodiment of the present invention may be made of a biocompatible silicon rubber material.

The strain relief portion of a preferred embodiment header assembly is adapted to facilitate a user visually verifying when a lead has been fully inserted into the header. For example, at least a portion of the strain relief portion may be substantially transparent for visually verifying when a particular portion of the lead is in juxtaposition therewith. Structure, such as a band or ring, is provided upon embodiments of the strain relief portion in order to provide a readily identifiable visual aid for determining when a lead is fully inserted.

It should be appreciated that an integral strain relief portion as provided according to embodiments of the present invention provides advantages in that there is no separate part to manufacture, inventory, track, include into a kit or other product package, etcetera. Moreover, manufacturing such a strain relief portion as a part of a header assembly allows a simple and relatively inexpensive lead assembly to be used, as compared to a lead assembly having an integrated strain relief as in the prior art, although installation of the lead assembly into the medical device is simplified.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated that the conception and specific embodiment disclosed may be readily utilized as a basis for modifying or designing other structures for carrying out the same purposes of the present invention. It should also be realized that such equivalent constructions do not depart from the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWING

For a more complete understanding of the present invention, reference is now made to the following descriptions taken in conjunction with the accompanying drawing, in which:

FIGURE 1 shows a prior art configuration in which a strain relief member is provided separate from a lead and corresponding medical device;

FIGURE 2 shows a prior art configuration in which a strain relief component is integral with a rigid lead plug assembly;

FIGURE 3 shows a medical device header assembly of an embodiment of the present invention having a strain relief portion integral therewith;

FIGURES 4A and 4B show an embodiment of the present invention with a lead inserted into a header assembly having a strain relief portion integral therewith;

FIGURE 5 shows a cross section view of a strain relief portion of a header assembly of an embodiment of the present invention; and

FIGURES 6-11 show alternative embodiments of a medical device header assembly having a strain relief portion integral therewith.

### DETAILED DESCRIPTION OF THE INVENTION

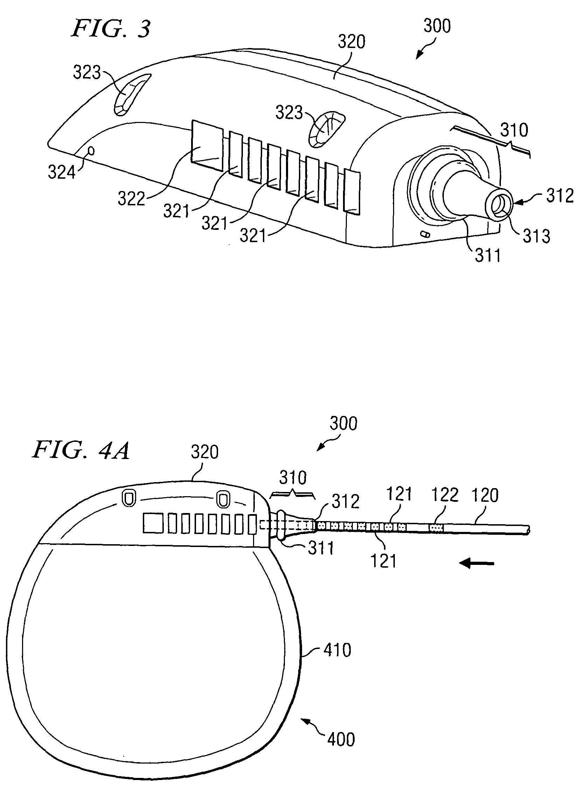

Directing attention to FIGURE 3, a medical device header assembly adapted according to an embodiment of the present invention is shown as header assembly 300. Header assembly 300 of the illustrated embodiment includes strain relief portion 310 and body portion 320. Strain relief portion 310 and body portion 320 of preferred embodiments, although providing separate and distinct functional aspects, are integrated as a single header assembly unit. Such an embodiment provides an inexpensive and relatively simple to manufacture solution, as well as eliminating costs and errors associated with collecting components into a kit or assembly, assembling components, etcetera.

Header assembly 300, and thus strain relief portion 310 and body portion 320, of a preferred embodiment is comprised of a resilient, biocompatible, material, such as silicone rubber (e.g., SILASTIC available from Dow Coming Corporation, Midland Michigan). Accordingly, header assembly 300 may be injection molded or otherwise cast as a monolithic structure from a suitable resilient material. According to embodiments of the invention, body portion 320 may comprise a material different than that of strain relief 310, although both are preferably formed as a unitary header assembly structure. For example, body portion 320 may comprise a material of more significant hardness (e.g., polycarbonate or polyurethane) than strain relief 310 (e.g., silicon rubber).

Body portion 320 is preferably adapted to receive an end of a lead assembly, such as lead 120 shown in FIGURE 1, and provide electrical interfacing between the lead and a medical device (not shown). Accordingly, body portion 320 of the illustrated embodiment includes cavities 321, disposed in alignment with electrodes of a corresponding lead when fully inserted, into which electrical contact assemblies may be disposed. For example, spring biased contact assemblies (not shown) may be disposed in each of cavities 321 to provide electrical contact with corresponding ones of electrodes 121 of lead 120 when inserted into a lead canal (not visible in FIGURE 3) internal to header assembly 300. The foregoing electrical contact assemblies preferably allow insertion of a lead end through the header assembly lead canal during installation while providing a reliable electrical contact when the lead is fully inserted into the lead canal. Cavity 322 of embodiments, also disposed in the illustrated embodiment in alignment with an electrode of a corresponding lead when fully inserted, may house a locking mechanism to securely hold the lead when fully inserted. For example, a set screw assembly (not shown) may be disposed in cavity 322 to provide a mechanical holding force preventing removal of the lead from the lead canal of header assembly 300.

Body portion 320 of the illustrated embodiment includes features in addition to the above described cavities. For example, body portion 320 as shown includes suture rings 323, facilitating suturing header assembly 300, and thus an attached medical device and lead, to living tissue, such as where the medical device comprises an implantable pulse generator of a neurostimulation system. Additionally, body portion 320 of the illustrated embodiment includes fill port 324, such as may be in communication with any or all of cavities 321 and 322 and/or the aforementioned lead canal. Fill port 324 may facilitate the introduction of material, such as a biocompatible adhesive, filler, or gel, to be injected into areas of header assembly 300 after insertion of a lead to prevent or discourage body fluids or other potential contaminants from infiltrating such areas.

It should be appreciated that body portion 320 is not limited to the particular combination of features and aspects described with respect to the illustrated embodiment. For example, body portion 320 may include more or fewer (including no) cavities, suture rings, and fill ports. Moreover, body portion 320 may include features and aspects in addition to or in the alternative to the foregoing. For example, body portion 320 may include one or more annular seal disposed within the lead canal (e.g., an "O" ring or sealing ring protruding from the lumen of the canal) to discourage entry of contaminants and/or to clean the surface of the lead as it is inserted into the lead canal. Body portion 320 may include a radio-opaque identification tag, radio frequency identification tag, or other marker to facilitate location of the header assembly and/or medical device after being implanted into a human body. Body portion 320 may include an antenna assembly internal thereto, such as may be coupled to electronics of a medical device to facilitate radio frequency communication and/or energy transfer between the medical device and an external device, such as a clinician programmer or an external controller.

Strain relief portion 310 is preferably adapted to receive an end of a lead assembly, such as lead 120 shown in FIGURE 1, and to prevent the lead from being tightly radiused or otherwise having excess strain exerted thereon when fully inserted into a lead canal of header assembly 300. Strain relief portion 310 of the illustrated embodiment includes orifice 312 at a distal end thereof, defining a beginning of the aforementioned lead canal, to accept an end of a lead. The lead canal associated with orifice 312 extends through strain relief portion 310 and into body portion 320 to allow insertion of a lead and placing electrodes thereof in juxtaposition with cavities 321 and 322.

Strain relief portion 310 of the illustrated embodiment includes annular seal 313 disposed in orifice 312 to discourage entry of contaminants and/or to clean the surface of the lead as it is inserted into the lead canal. Although annular seal 313 may comprise a component separate from the material of strain relief portion 310, such as an "O" ring disposed in a detent within orifice 312, preferred embodiments provide an annular seal configuration which is integral to strain relief portion 310. For example, a sealing ring may be formed by the material of strain relief portion 310 protruding from the lumen of orifice 312 to thereby form annular seal 313, where strain relief portion 310 is comprised of a resilient material. Such an embodiment provides an inexpensive and relatively simple to manufacture solution, as well as eliminating costs and errors associated with collecting components into a kit or assembly, assembling components, etcetera.

Strain relief portion 310 of the illustrated embodiment includes alignment indicator 311 to facilitate determining when a lead has been fully inserted into header assembly 300. For example, although an end of the lead engaging a stop at the end of the lead canal, a lead electrode engaging a portion of a locking mechanism disposed in cavity 322, and/or electrodes engaging a portion of electrical contact assemblies disposed in cavities 321 may provide tactile feedback, alignment indicator 311 of a preferred embodiment provides visual feedback with respect to the proper insertion of a lead into header assembly 300. According to an embodiment of the present invention, alignment indicator 311 comprises a transparent area of strain relief portion 310. A user may view at least a portion of a lead through alignment indicator 311 as the lead is being inserted into header assembly 300 and determine when the lead is fully or otherwise properly inserted.

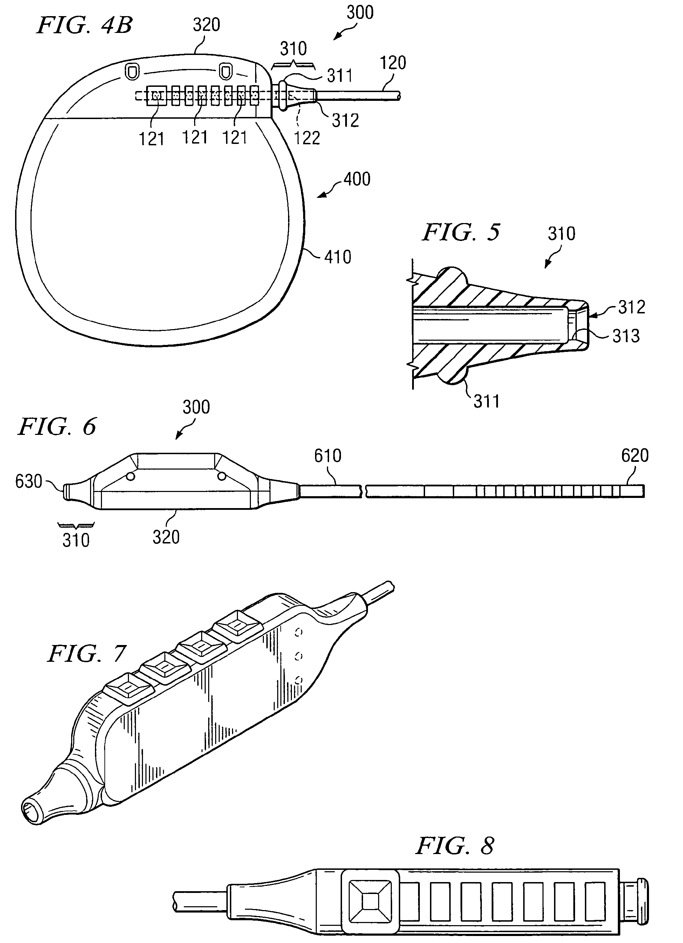

Directing attention to FIGURES 4A and 4B, an embodiment of the present invention is shown with a lead inserted into header assembly 300. Specifically, lead 120 is shown inserted into header assembly 300 disposed upon medical device 410. Medical device 410 may comprise an implantable pulse generator, such as a part of the GENESIS implantable pulse generator available from Advanced Neuromodulation Systems, Inc. of Plano Texas (the assignee of the present application), whereby medical device 410, header assembly 300, and lead 120 provide neurostimulation system 400.

As shown in FIGURE 4A, lead 120 is inserted into strain relief portion 310 and body portion 320 of header assembly 300 through orifice 312. Insertion handle 122, such as may comprise a band of relatively rigid material disposed integral to or on an area of lead 120, provides a relatively rigid portion of an otherwise flexible lead to facilitate insertion of lead 120 into header assembly 300. As lead 120 proceeds into header assembly 300, different portions of lead 120 become visible through alignment indicator 311. As lead 120 of a preferred embodiment becomes fully inserted into header assembly 300, insertion handle 122, or a portion thereof, becomes visible within alignment indicator 311 as shown in FIGURE 4B. For example, proper insertion of lead 120 into header assembly 300 may be indicated when an edge of insertion handle 122 (e.g., a front or rear edge) becomes visibly aligned with an edge of alignment indicator 311 (e.g., a front or rear edge). Additionally or alternatively, proper insertion of lead 120 into header assembly 300 may be indicated with all of insertion handle 122 is visible within alignment indicator 310 or when insertion handle 122 is fully visible within alignment indicator 310. Accordingly, the rigid portion of lead 120 associated with insertion handle 122 is disposed well within strain relief portion 310, thereby disposing a flexible portion of lead 120 toward the distal end and out of orifice 312 to facilitate flexing or bending of lead 120 (as controlled by strain relief portion 310) at the interface of the lead and medical device. Upon determining that lead 120 is fully or otherwise properly inserted into header assembly 300, steps may be taken to hold the lead in the desired position, such as by engaging a set screw of the aforementioned locking mechanism and/or by tying a suture around strain relief portion 310 to increase friction contact with the lead.

Strain relief portion 310 of embodiments of the invention may be adapted to further facilitate determining when lead 120 is fully or properly inserted into header assembly 300. For example, areas of strain relief portion 310 may be less light transmissive than alignment indicator 311 to facilitate ready appreciation of a particular portion of lead 120 becoming visible within alignment indicator 311. According to embodiments of the invention, areas of strain relief portion 310 are opaque whereas alignment indicator 311 is transparent. The foregoing less light transmissive or opaque areas may be provided by a matt finish resulting from the surface of a mold used in injection molding header assembly 300 being relatively coarse, such as may result from an electronic machining process used in manufacturing the mold. The more light transmissive or transparent areas may be provided by a gloss finish resulting from polishing the surface of a mold used in injection molding header assembly 300 corresponding to alignment indicator 311.

Alignment indicator 311 may, additionally or alternatively, be adapted to provide one or more optical characteristics to further facilitate determining when lead 120 is fully or properly inserted into header assembly 300. For example, alignment indicator 311 may present a cross section having characteristics of a lens, as shown in FIGURE 5, to optimize visualizing when a particular portion of the lead is disposed thereunder.
According to embodiments of the invention, a lens characteristic of alignment indicator 311 magnifies lead 120 such that insertion handle 122 is highly visible when disposed in juxtaposition with alignment indicator 311.

It can be appreciated, from the cross section view of FIGURE 5, that the walls of strain relief portion 310 of the illustrated embodiment provide a tapered profile. This tapered profile provides increasing resistance to bending from the distal end (the end with orifice 312) to the proximal end (the end interfaced with body portion 320) and thus resists tightly radiusing lead 120 at the interface with the medical device.

Although particular embodiments have been described above, it should be appreciated that the concepts of the present invention are not limited to the embodiments described herein. For example, although use of alignment indicator 311 to determine full or proper insertion of a lead has been described with reference to an insertion handle, the present invention is not limited to use with such an insertion handle. Other demarcations upon a lead being inserted into header assembly 300 may be utilized according to embodiments of the invention. For example, a mark or ring on a lead, a surface feature on a lead, and/or the like may be used according to embodiments of the invention. Similarly, alignment indicator 311 of embodiments need not comprise a transparent area. For example, alignment indicator 311 may comprise a mark or ring (e.g., printed mark or ring), perhaps adjacent to a light transmissive (e.g., transparent or translucent) area of strain relief portion 310, for referencing when a portion of the lead is aligned therewith. However, preferred embodiments of the invention do not implement marks added to one or more components of system 400 solely for use in determining full or proper insertion of the lead because such marks may involve additional manufacturing steps (potentially adding time and costs to the manufacturing process) and the material used in making the marks may require biocompatibility testing etcetera. Accordingly, preferred embodiments of the present invention implement a configuration of alignment indicator 311 which is fully integral to strain relief portion 310, providing one or more optical properties as described above.

Embodiments of the present invention may implement techniques for determining full or proper insertion of a lead in addition to or in the alternative to the aforementioned alignment indicator. For example, a lead stop may be employed within body portion 320 to provide tactile feedback that the lead has been fully inserted.

Although an embodiment of header assembly 300 has been described above with reference to a medical device comprising a pulse generator, header assemblies of the present invention may be utilized with respect to a variety of medical devices and medical device configurations. For example, header assembly 300 of the embodiment of FIGURE 6 is adapted for disposing upon medical device 610, such as may comprise a lead extension, configured differently than medical device 410 discussed above. Medical device 610 of embodiments provides a lead extension comprising end 620 for interfacing with another medical device, such as medical device 410 of FIGURE 4A, and end 630 having header assembly 300 disposed thereat. Header assembly 300 provides a receiver to accept an end of a lead, such as lead 120, using body portion 320. Strain relief portion 310 preferably prevents a lead coupled thereto from being tightly radiused or otherwise having excess strain exerted thereon. Body portion 320 may be relied upon to provide similar strain relief with respect to a portion of medical device 610. Additionally or alternatively, header assembly 300 may be provided with an additional strain relief portion, similar in configuration to strain relief portion 310, to provide strain relief with respect to a portion of medical device 610.

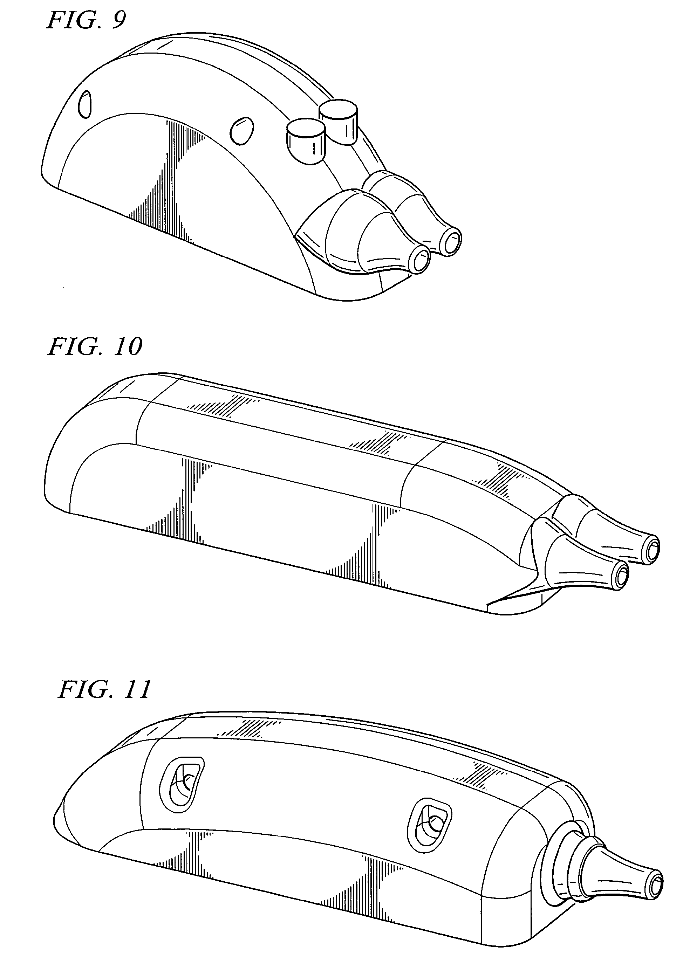

FIGURES 7-11 show alternative embodiment configurations of medical device header assemblies of the present invention. FIGURES 7 and 8 show alternative embodiments configured for use with medical devices such as lead extensions. FIGURES 9, 10, and 11 show alternative embodiments configured for use with medical devices such as implantable pulse generators. It should be appreciated that the foregoing embodiments are not exhaustive of the various configurations which may embody the concepts of the present invention, but rather are illustrative of embodiments which may be implemented.

Although the present invention and its advantages have been described in detail, it should be understood that various changes, substitutions and alterations can be made herein without departing from the invention as defined by the appended claims. Moreover, the scope of the present application is not intended to be limited to the particular embodiments of the process, machine, manufacture, composition of matter, means, methods and steps described in the specification. As one will readily appreciate from the disclosure, processes, machines, manufacture, compositions of matter, means, methods, or steps, presently existing or later to be developed that perform substantially the same function or achieve substantially the same result as the corresponding embodiments described herein may be utilized. Accordingly, the appended claims are intended to include within their scope such processes, machines, manufacture, compositions of matter, means, methods, or steps.

## Claims

1. A system comprising:
a medical device header assembly, said medical device header assembly comprising:
a body portion for accepting an end of a lead; and
a strain relief portion for providing strain relief with respect to said lead;
wherein said body portion and said strain relief portion are permanently integrated to comprise said header assembly.

2. The system of claim 1, wherein said strain relief portion is comprised of a resilient material.

3. The system of claim 1, wherein said body portion and said strain relief portion are cast as a monolithic structure.

4. The system of claim 1, wherein said strain relief portion comprises an alignment indicator providing a user with a visual indication that said lead is inserted into said header assembly to a predetermined position.

5. The system of claim 4, wherein said alignment indicator is disposed near an end of said strain relief portion which interfaces with said body portion.

6. The system of claim 4, wherein said alignment indicator comprises a transparent area of said strain relief portion, and wherein said transparent area is adapted to optimize visualizing when a particular portion of the said is disposed thereunder.

7. The system of claim 6, wherein said transparent area is shaped to provide lens functionality.

8. The system of claim 4, wherein said alignment indicator comprises an area of said strain relief portion which is more light transmissive than an area of said strain relief portion adjacent to said alignment indicator.

9. The system of claim 4, wherein said visual indication that said lead is inserted into said header assembly to a predetermined position comprises at least a portion of a surface feature of said lead being disposed in juxtaposition with said alignment indicator.

10. The system of claim 1, wherein said strain relief portion comprises:
an annular seal disposed at a lead orifice of the strain relief portion.

11. The system of claim 1, wherein a medical device with which said header assembly is used comprises an implantable pulse generator of an electrical stimulation system.

12. The system of claim 1, wherein a medical device with which said header assembly is used comprises a lead extension.

13. A method comprising:
providing a medical device header assembly having a strain relief portion integral with a body portion;
inserting a lead into said device header assembly until said lead is inserted to a desired position; and
holding the lead in said desired position.

14. The method of claim 13, wherein said providing a header assembly comprises:
monolithically forming said strain relief portion and said body portion.

15. The method of claim 13, wherein said holding the lead in said desired position comprises at least one of engaging a locking mechanism and tightening a suture around said strain relief portion.

16. The method of claim 13, further comprising:
determining said lead is inserted to said desired position by visually determining when a particular part of said lead is in juxtaposition with an alignment indicator of said strain relief portion.

17. A system for providing strain relief to a lead when interfaced with a medial device, said system comprising:
a medical device header assembly having a strain relief portion; and
a medical device, said medical device header assembly being attached to said medical device.

18. The system of claim 17, wherein said strain relief portion is formed integral with said medical device header assembly.

19. The system of claim 17, wherein a portion of said lead inserted into said medical device header assembly is flexible.

20. The system of claim 17, further comprising:
a lead, said lead inserted into said medical device header assembly through said strain relief portion to a position that at least a portion of a particular feature of said lead is visually in juxtaposition with at least a portion of an alignment indicator integral with said strain relief portion.
